# EUROPÄISCHE PATENTSCHRIFT

(11) **EP 1 857 129 B1**
(45) Veröffentlichungstag und Bekanntmachung des Hinweises auf die Patenterteilung: **11.03.2009**
(21) Anmeldenummer: 07405135.0
(22) Anmeldetag: 08.05.2007
(51) Int. Cl.: A61M 5/142

(54) **Adaptervorrichtung für das Ankleben eines medizinischen Geräts an die Hautoberfläche**
Adapter device for sticking a medical instrument to the skin surface
Dispositif d'adaptateur pour le collage d'un appareil médical à la surface de la peau

(30) Priorität: 19.05.2006 CH 8152006
(43) Veröffentlichungstag der Anmeldung: 21.11.2007
(73) Patentinhaber: Roche Diagnostics GmbH, 68305 Mannheim (DE)
(72) Erfinder: Kaufmann, Heiner, Bern 3008 (CH); Kühni, Florian, 4938 Rohrbach (CH); Remde, Axel, 3432 Lützelflüh-Goldbach (CH); Studer, Gérald, 1950 Sion (CH)
(74) Vertreter: Rüfenacht, Philipp Michael

(56) Entgegenhaltungen:
- WO-A-97/21457
- WO-A-03/028784
- WO-A-20/04098684

## Beschreibung

Die Erfindung betrifft eine Adapter- oder Hilfsvorrichtung welche dazu geeignet ist die manuelle Befestigung eines mit Klebemitteln versehenen portablen, medizinischen Gerätes an die Hautoberfläche eines menschlichen oder tierischen Körpers sicher, ergonomisch und positionsgenau zu ermöglichen.

Dank dem technischen Fortschritt in Mikroelektronik und -mechanik sind portable, medizinische Geräte mit stetig kleineren Bauvolumina und geringeren Eigengewichten realisierbar. Dadurch wird, mit den Vorteilen eines erhöhten Tragekomforts und besserer Diskretion, eine direkte Befestigung an der Hautoberfläche eines menschlichen oder tierischen Körpers möglich.

Portable medizinische Geräte in Form von Infusionsvornchtungen, welche direkt an der Hautoberfläche befestigt werden und somit ein diskretes und komfortables Tragen ermöglichen, werden beispielsweise zur Behandlung von Diabetespatienten eingesetzt. Im Idealfall klebt eine solche körpergetragene Infusionsvorrichtung für 4-5 Tage am Körper und ist wasserdicht. Der Austausch der Infusionsvorrichtung, unter wechselnder Lokalisation des Kanüleneinstiches, wird vom Patienten eigenhändig vorgenommen.

Weitere hautbefestigbare Geräte sind im Anwendungsfeld der kontinuierlichen Erfassung von Blutzuckermesswerten im menschlichen Körper bekannt. Ein Glukosemessensor welcher am körperaussenseitig verbleibenden Gehäuseteil angebracht ist, wird zur Ermittlung des Blutzuckerwertes subkutan ins Körpergewebe eingeführt.

Die Befestigung solcher medizinischen Geräte stellt eine Reihe grundsätzlicher Anforderungen, damit ein sicherer Halt, ein guter Tragekomfort und eine positionsgenaue Platzierung gewährleistet werden können.

Für die Befestigung an der Hautoberfläche muss eine sichere Verbindung zwischen Körper und medizinischem Gerät während der Gebrauchsdauer gewährleistet sein. Selbstklebende Pflastermaterialien erlauben eine genügende Klebefähigkeit während der Verweildauer des medizinischen Gerätes auf der Hautoberfläche, sofern die Klebefläche des medizinischen Gerätes beim Aufbringvorgang mit einer ausreichenden Flächenpressung mit der Hautoberfläche in Verbindung gebracht worden ist. Eine gute Handhabbarkeit des aufzubringenden Gerätes während dieses Aufbringvorganges ist dazu unabdingbar. Bei miniaturisierten Geräten ist diese Anforderung oftmals schwierig zu realisieren.

Für eine sichere Befestigung und eine komfortable Trageweise sind die durch Bekleidung und Schwerkraft erzeugten Kippmomente so gering wie möglich zu halten. Durch die konstruktive Ausgestaltung mit einer möglichst geringen Bauhöhe kann dieser Forderung nachgekommen werden. Für das Aufbringen des medizinischen Gerätes erweist sich jedoch eine solche, wenig griffige Formgestalt als nachteilig. Ergonomische ausgestaltete Griffmittel welche eine sichere Handhabung des medizinischen Gerätes während des Aufbringvorganges ermöglichen, erweisen sich während der Gebrauchsdauer als störend.

Durch die plattige Formgestalt eines an der Haut befestigten medizinischen Gerätes, beispielsweise einer Insulinpumpe, wird dem Patienten beim Aufbringvorgang die notwendige Sicht auf die Einstechkanüle bzw. des einzuführenden Sensors verdeckt. Eine sichere und positionsgenaue Fixierung des medizinischen Gerätes auf der Hautoberfläche wird erschwert oder gar verunmöglicht.

Im Stand der Technik sind am Körper befestigbare Infusionspumpen bekannt, welche einen modularen Aufbau aufweisen. Eine modulare Ausgestaltung einer solchen Infusionspumpe ist in der Patentschrift DE 198 21 723 C2 beschrieben.

Des Weiteren sind im Stand der Technik Ausführungsformen bekannt, welche Aufbring- oder Adaptervorrichtungen mit Klebemittel zwischen medizinischem Gerät und Hautoberfläche offenbaren. Patentschrift WO/2004/060436 A2 zeigt eine Adapterplatte welche geeignet ist, eine an der Hautoberfläche anbringbare Infusionspumpe für den Einmalgebrauch, mittels unterschiedliche ausgestalteten Klebeflächen zu befestigen. US 2004/0116866 A1 beschreibt eine am menschlichen oder tierischen Körper anklebbare Infusionspumpe mit mehrteiligen Klebefeldern, welche optimale Kraftschlussverhältnisse bei unterschiedlichen Belastungen erlauben. Im Bereich der Infusionsstelle sind mehrere Klebesegmente mit unterschiedlichen Klebeeigenschaften und Steifigkeiten offenbart.

Die WO 2004/098684 A1 (Novo Nordisk) beschreibt ein medizinisches Gerät, welches auf der Haut eines Patienten aufgeklebt werden kann, mit einem Anbringungsteil und einem Handhabungsteil. Der Handhabungsteil bildet dabei ein Gehäuse für den Anbringungsteil, bevor dieser auf der Haut angebracht wird. Der Anbringungsteil und der Handhabungsteil können voneinander getrennt werden. Es wird weiter offenbart, dass sowohl der Anbringungsteil als auch der Handhabungsteil über einen Verschwenkmechanismus verfügen, wobei die entsprechende Verschwenkbarkeit beim Setzen einer Nadel ausgenutzt wird. Der Verschwenkmechanismus wird durch ein lösbares Fixierungsmittel in einer Ausgangsstellung gehalten. Das Fixierungsmittel umfasst dazu eine Sollbruchstelle, die ein Verschwenken von der fixierten Ausgangsstellung in eine Gebrauchsstellung nach Überschreiten eines auf den Verschwenkmechanismus wirkenden Drehmoments erlaubt.

Die vorliegende Erfindung hat sich zur Aufgabe gemacht das manuelle Aufbringen eines an die Hautoberfläche eines menschlichen oder tierischen Körpers zu befestigenden medizinischen Gerätes mittels einer Adaptervorrichtung sicher, ergonomisch und positionsgenau zu ermöglichen. Dies wird mit der Vorrichtung gemäss Anspruch 1 erreicht.

Die Adaptervorrichtung ist im Ausgangszustand mit dem medizinischen Gerät fest verbunden und ist mit einer ergonomisch ausgebildeten Griffvorrichtung ausgestattet damit das miniaturisierte Gerät gut, stabil und griffig in der Hand des Anwenders gehalten werden kann. Die feste Verbindung zwischen Adaptervorrichtung und medizinischem Gerät kann sowohl kraft- als auch formschlüssig realisiert sein. Als Ausgangszustand wird der Zustand bezeichnet, aus welchem der Aufbringvorgang des medizinischen Gerätes auf die Hautoberfläche erfolgt und bei welchem der Verschwenkteil mit einem Fixierungsmittel in einem bestimmten Winkel zum Aufsetzteil gesichert ist.

Die Verschwenkvorrichtung bewirkt eine Gliederung der Adaptervorrichtung und des fest verbundenen medizinischen Gerätes in ein Aufsetz- und ein Verschwenkteil. Als Aufsetzteil wird der Teil bezeichnet, der zuerst auf die Hautoberfläche aufgesetzt bzw. angeklebt wird. Der Verschwenkteil ragt im Ausgangszustand in einem Winkel von der Grundfläche derart ab, dass die Klebeunterseite des Verschwenkteiles nicht mit der Hautoberfläche in Berührung kommt. Der zwischen Aufsetz- und Verschwenkteil befindliche Verschwenkmechanismus erlaubt eine Bewegung von der Auslenkstellung in die Gebrauchsstellung. In der Gebrauchsstellung liegen Aufsetz- und Verschwenkteil annähernd planar auf der Körperoberfläche auf und gewährleisten über die Klebeflächenverbindungen eine sichere Befestigung des medizinischen Gerätes.

Die erfinderische Ausführungsform setzt eine gewisse Verformbarkeit des medizinischen Gerätes voraus. Liegt sie nicht vor, muss bei Geräten mit einem starren oder biegesteifen Gehäuse die notwendige Flexibilität, beispielsweise mittels einer gelenkigen Verbindung zwischen Aufsetz- und Verschwenkteil, konstruktiv herbeigeführt werden.

Mit der Griffvorrichtung, welche sich distal auf dem Aufsetzteil befindet, wird eine ergonomische und griffige Handhabung des medizinischen Gerätes erreicht. Dank der ergonomischen Ausgestaltung kann das Aufpressen der Klebefläche auf die Hautoberfläche besser vorgenommen werden bzw. die notwendige Flächenpressung für eine sichere Klebeverbindung gewährleistet werden.

In einer bevorzugten Ausführungsform ist die Verschwenkvorrichtung, mittels einer in Auslenkstellung einrastbaren Gelenkverbindung, die manuell durch einen Auslösemechanismus gelöst werden kann, realisiert. Dadurch kann der Adapter Platz sparend verpackt und ausgeliefert werden. Vor der Applikation auf das Hautgewebe wird der Verschwenkteil in die Auslenkstellung gebracht, wo er einrastet. Das Verschwenken in die Gebrauchsstellung erfolgt mittels Betätigung des manuellen Auslösemechanismus oder bei einer Sollbruchstelle durch Überschreitung eines an der an der Verschwenkvorrichtung manuell erzeugten Bruchmomentes.

In einer bevorzugten Ausführungsform ist die Verschwenkvorrichtung als gelenkig ausgebildete Verbindung zwischen Aufbring- und Verschwenkteil ausgeführt. Das konstruktiv als Sollbruchstelle ausgestaltete Fixierungsmittel ermöglicht die gewünschte Verschwenkbewegung nach Erzeugen eines ausreichend grossen, manuell erzeugten Bruchmomentes.

In einer weiteren bevorzugten Ausführungsform kann die Sollbruchstelle durch eine manuell leicht lösbare Verbindung, beispielsweise einem quer liegenden Steg oder einer Lasche, zwischen Verschwenk- und Aufbringteil realisiert sein.

Eine weitere bevorzugte Ausführungsform, bei welcher die gelenkige Verbindung eine Fixierung in einer Rastposition bewirkt und sich nach einem Überschreiten eines Momentes löst, erlaubt aus einer beliebigen Ausgangslage eine Ankröpfung in die Ausgangslage.

Der mit dem Adapter während des Befestigungsvorganges verbundene Griffabschnitt dient dazu, die kleine Pumpe gut und stabil in der Hand zu halten, womit das Stechen der Nadel erleichtert wird.

Um eine möglichst flache Formgestalt des medizinischen Gerätes zu erreichen, wird der Griffabschnitt nach der Applizierung vom medizinischen Gerät entfernt oder derart ausgestaltet, dass die Griffvorrichtung nach Gebrauch vom Gerätegehäuse aufgenommen werden kann.

In einer weiteren bevorzugten Ausführungsform wird ein medizinisches Gerät mit einer Sensoreinrichtung, mit welchem ein Sensorelement in das Körpergewebe eingeführt wird, körperbefestigt.

Im Folgenden werden Ausführungsformen der Erfindung anhand einiger Figuren näher erläutert.

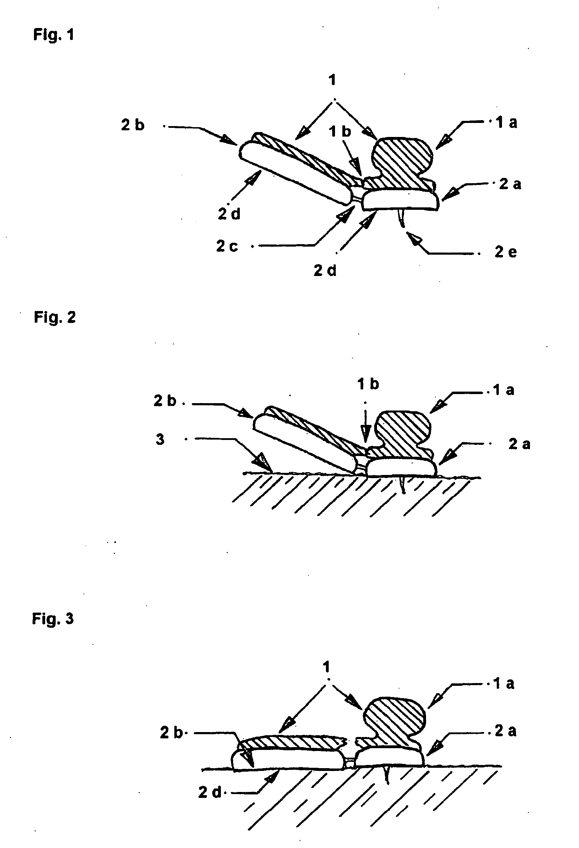

Figur 1 zeigt eine erfindungsgemässe Ausführung eines Adapters (1), welcher auf einem medizinischen Gerät (2a,2b) angebracht ist. Im gezeigten Ausführungsbeispiel handelt es sich beim medizinischen Gerät um eine an der Hautoberfläche befestigte Insulinpumpe mit integriertem Katheterkopf. Der distal über dem Aufsetzteil (2a) angeordnete Griff (1a) erlaubt dem Patienten die Einstechkanüle (2e) ergonomisch und mit gutem Griff in das Körpergewebe einzubringen. Durch die von der Adaptervorrichtung herbeigeführte Abkröpfung bzw. Auslenkung des Verschwenkteiles (2b) gegenüber der Hautoberfläche wird dem Anwender eine gute Sicht auf die Einstichstelle gewährt, was eine positionsgenaue Fixierung ermöglicht. Dank der guten Handhabbarkeit und der fixierten Ausgangsstellung des Verschwenkteiles (2b) kann eine sichere Anpressung des Aufsetzteiles (2a) auf der Hautoberfläche vorgenommen werden.

Figur. 2 zeigt eine Infusionspumpe, bei welcher der Aufsetzteil (2a) bereits am Körper angeklebt worden ist. Aus diesem Zustand erfolgt das Einschwenken des Verschwenkteiles (2b) in die Gebrauchsstellung durch ein manuell herbeigeführtes Versagen der Fixierung (1b), welche im vorliegenden Falle konstruktiv als Sollbruchstelle ausgebildet ist

Fig. 3 zeigt Infusionspumpe und Adapter in Gebrauchsstellung nach einem Herunterklappen aus der Auslenkstellung. Aufsetz- (2a) und Verschwenkteil (2b) liegen annähernd planar auf der Körperoberfläche auf und sind mittels Klebeflächen mit der Hautoberfläche in Verbindung gebracht.

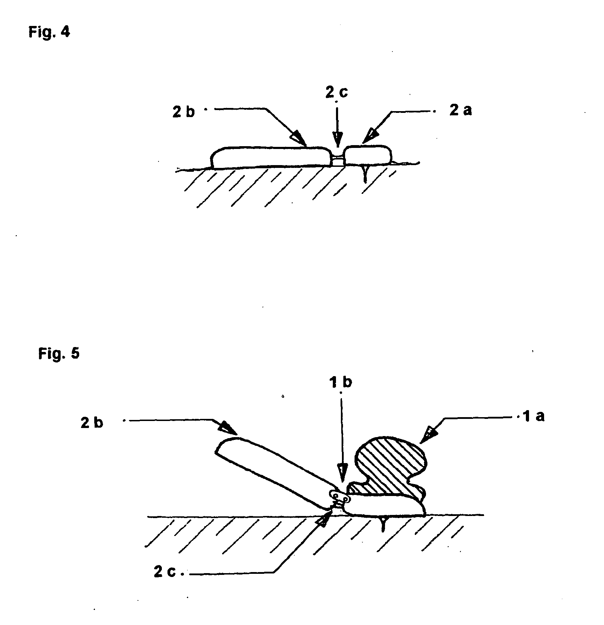

Fig. 4 zeigt ein medizinisches Gerät in Form einer Insulinpumpe in der Gebrauchsstellung nach Entfernung des Adapters. Die Pumpenteile (2a,2b) sind flexibel miteinander verbunden und passen sich an die Hautoberfläche an. Ein kurzer, in der gelenkigen Verbindung integrierter, Flüssigkeitspfad verbindet die beiden Teile.

Fig. 5 zeigt eine erfindungsgemässe Ausführung eines Adapters bei welchem der Verschwenkmechanismus (2c) in das medizinischen Gerät integriert ist. Das Fixierungsmittel (1b) mit einer Sollbruchstelle, ist zwischen Verschwenk- und Absetzteil angebracht. Der Adapter kann damit einstückig, mit dem Haltegriff (1a) distal über dem Aufsetzteil, ausgeführt sein. Auf einen Adapterabschnitt auf dem Verschwenkteil (2b) kann verzichtet werden

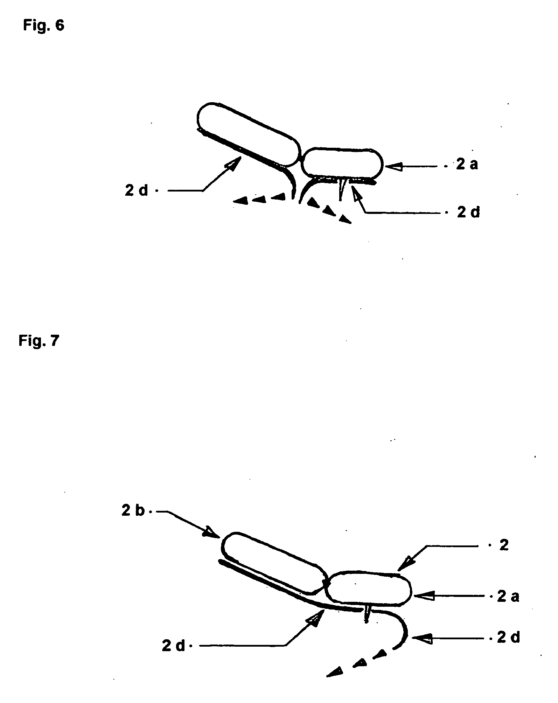

Fig. 6 zeigt eine Ausführungsform mit Klebemitteln (2d) auf der Geräteunterseite des Verschwenkteils (2b) und des Aufsetzteils (2a). Durch die gelenkige Ausgestaltung des medizinischen Gerätes mit dem Verschwenkmechanismus (2c) wird eine Anpassung an eine nicht planare Hautoberfläche gewährleistet.

Fig. 7 zeigt eine Ausführungsform eines sich über die Geräteunterseite erstreckenden einstückigen Klebemittels (2d).

## Patentansprüche

1. Adaptervorrichtung für das manuelle Aufbringen eines mit einer Klebevorrichtung (2d) vorbereiteten medizinischen Gerätes (2a,2b,2c) an die Hautoberfläche (3) eines menschlichen oder tierischen Körpers, umfassend einen Adapter (1) und ein medizinisches Gerät (2), wobei der im Ausgangszustand mit dem medizinischen Gerät eine Einheit bildende Adapter (1) eine Griffvorrichtung (1a) aufweist und wobei die Adaptervorrichtung mit einem Verschwenkmechanismus (2c) versehen ist, welcher das medizinische Gerät in einen Aufsetzteil (2a) und einen Verschwenkteil (2b) gliedert, **dadurch gekennzeichnet, dass** sowohl der Aufsetzteil (2a) als auch der Verschwenkteil (2b) eine Klebeunterseite zum Aufbringen des Gerätes (2a, 2b, 2c) an die Hautoberfläche (3) aufweisen.

2. Adaptervorrichtung nach Anspruch 1, **dadurch gekennzeichnet, dass** der Verschwenkmechanismus (2c) eine Auslenkung des Verschwenkteils (2b) um einen Winkel ermöglicht, welcher, bei auf der Hautoberfläche (3) angebrachtem Aufsetzteil (2a), in der ausgelenkten Stellung einen Klebekontakt des Verschwenkteiles (2b) mit der Hautoberfläche verhindert.

3. Adaptervorrichtung nach Anspruch 1 oder 2, **dadurch gekennzeichnet, dass** der Verschwenkmechanismus (2c) mit mindestens einem Fixierungsmittel (1b) kooperiert, welches nach einer Lösung der Fixierung ein Einklappen des Verschwenkteiles (2b) von einer Auslenkstellung in eine Gebrauchsstellung ermöglicht.

4. Adaptervorrichtung nach Anspruch 3, **dadurch gekennzeichnet, dass** der Verschwenkmechanismus (2c) konstruktiv als Gelenk mit einer Rastposition, welches ein Fixierungsmittel (1 b) bildet, ausgestaltet ist.

5. Adaptervorrichtung nach Anspruch 3 oder 4, **dadurch gekennzeichnet, dass** das Fixierungsmittel (1b) eine Sollbruchstelle enthält, welche nach Überschreiten eines auf den Verschwenkmechanismus (2c) wirkenden Drehmomentes ein Verschwenken von der fixierten Auslenkstellung in die Gebrauchsstellung erlaubt.

6. Adaptervorrichtung nach Anspruch 3, **dadurch gekennzeichnet, dass** das Fixierungsmittel (1b) eine Sollbruchstelle aufweist, welche bei Überschreiten einer auf die Sollbruchstelle wirkender Scherkraft aufgetrennt wird und ein Verschwenken von der fixierten Auslenkstellung in die Gebrauchsstellung bewirkt.

7. Adaptervorrichtung nach Anspruch 3, **dadurch gekennzeichnet, dass** das Fixierungsmittel (1b) durch einen entfernbaren Steg gebildet wird, welcher, nach dessen Entfernung, ein Verschwenken des Verschwenkteiles (2b) von der fixierten Auslenkstellung in die Gebrauchsstellung bewirkt.

8. Adaptervorrichtung nach einem der vorangehenden Ansprüche, **dadurch gekennzeichnet, dass** der Adapter im Ausgangszustand kraftschlüssig mit dem medizinischen Gerät (2a,2b,2c) verbunden ist.

9. Adaptervorrichtung nach einem der vorangehenden Ansprüche, **dadurch gekennzeichnet, dass** der Adapter (1) nach erfolgtem Befestigungsvorgang mindestens teilweise vom medizinischen Gerät (2a,2b,2c) entfernt werden kann.

10. Adaptervorrichtung nach einem der Ansprüche 1 bis 8, **dadurch gekennzeichnet, dass** die Griffvorrichtung (1a) nach erfolgtem Befestigungsvorgang vom medizinischen Gerät aufgenommen werden kann.

11. Adaptervorrichtung nach einem der Ansprüche 1 bis 10, **dadurch gekennzeichnet, dass** der Verschwenkmechanismus (2c) in der Adaptervorrichtung integriert ist, falls ein medizinisches Gerät mit geringer Biegesteifigkeit vorliegt.

12. Adaptervorrichtung nach einem der Ansprüche 1 bis 10, **dadurch gekennzeichnet, dass** der Verschwenkmechanismus (2b) im medizinischen Gerät integriert ist.

13. Adaptervorrichtung nach einem der vorangehenden Ansprüche, **dadurch gekennzeichnet, dass** die auf der Geräteunterseite angebrachten Klebemittel (2d) des Aufsetzteiles (2a) und des Verschwenkteiles (2b) sich unabhängig voneinander als Klebestreifen abreissen lassen.

14. Adaptervorrichtung nach einem der vorangehenden Ansprüche, **dadurch gekennzeichnet, dass** die auf der Geräteunterseite angebrachten Klebemittel (2d) des Aufsetzteiles (2a) und des Verschwenkteiles (2b) einen zusammenhängenden Klebestreifen bilden.

15. Adaptervorrichtung nach einem der vorangehenden Ansprüche, **dadurch gekennzeichnet, dass** das medizinische Gerät (2a,2b,2c) eine am Körper befestigbare Verabreichungsvorrichtung zur medizinischen Verabreichung einer flüssigen Substanz ist.

16. Adaptervorrichtung nach einem der vorangehenden Ansprüche, **dadurch gekennzeichnet, dass** das medizinische Gerät eine am menschlichen Körper befestigbare Insulinpumpe ist.

17. Adaptervorrichtung nach einem der vorangehenden Ansprüche, **dadurch gekennzeichnet, dass** das medizinische Gerät eine an der Hautoberfläche befestigbare Vorrichtung mit mindestens einem Sensor zur Ermittlung mindestens eines Körperzustandes ist.

## Claims

1. Adapter device with which a medical appliance (2a, 2b, 2c) prepared with an adhesive arrangement (2d) can be applied manually to the skin surface (3) of a human or animal body, comprising an adapter (1) and a medical appliance (2), where the adapter (1) forms a unit with the medical appliance in the starting state and has a grip device (1a), and where the adapter device is provided with a pivot mechanism (2c) that divides the medical appliance into an attachment part (2a) and a pivot part (2b), **characterized in that** both the attachment part (2a) and also the pivot part (2b) have an adhesive underside for mounting the appliance (2a, 2b, 2c) on the skin surface (3).

2. Adapter device according to Claim 1, **characterized in that** the pivot mechanism (2c) permits a deflection of the pivot part (2b) about an angle which, when the attachment part (2a) is applied to the skin surface (3), prevents an adhesive contact between the pivot part (2b) and the skin surface in the deflected position.

3. Adapter device according to Claim 1 or 2, **characterized in that** the pivot mechanism (2c) cooperates with at least one fixing means (1b) which, after the fixing has been released, allows the pivot part (2b) to be folded in from a deflection position to a use position.

4. Adapter device according to Claim 3, **characterized in that** the pivot mechanism (2c) is constructed as a hinge which has a locked position and forms a fixing means (1b).

5. Adapter device according to Claim 3 or 4, **characterized in that** the fixing means (1b) comprises a predetermined break point which permits a pivoting movement from the fixed deflection position to the use position after a torque acting on the pivot mechanism (2c) is exceeded.

6. Adapter device according to Claim 3, **characterized in that** the fixing means (1b) has a predetermined break point which is severed, when a shearing force acting on the predetermined break point is exceeded, and causes a pivoting movement from the fixed deflection position to the use position.

7. Adapter device according to Claim 3, **characterized in that** the fixing means (1b) is formed by a removable web which, after it has been removed, causes a pivoting movement of the pivot part (2b) from the fixed deflection position to the use position.

8. Adapter device according to one of the preceding claims, **characterized in that** the adapter, in the starting state, is connected with a force-fit to the medical appliance (2a, 2b, 2c).

9. Adapter device according to one of the preceding claims, **characterized in that** the adapter (1) can be at least partially removed from the medical appliance (2a, 2b, 2c) when the securing procedure is completed.

10. Adapter device according to one of Claims 1 to 8, **characterized in that** the grip device (1a) can be detached from the medical appliance when the securing procedure is completed.

11. Adapter device according to one of Claims 1 to 10, **characterized in that** the pivot mechanism (2c) is integrated in the adapter device if a medical appliance with low flexural strength is present.

12. Adapter device according to one of Claims 1 to 10, **characterized in that** the pivot mechanism (2b) is integrated in the medical appliance.

13. Adapter device according to one of the preceding claims, **characterized in that** the adhesives (2d) applied to the underside of the appliance, on attachment part (2a) and pivot part (2b), can be torn off independently of one another as adhesive strips.

14. Adapter device according to one of the preceding claims, **characterized in that** the adhesives (2d) applied to the underside of the appliance, on attachment part (2a) and pivot part (2b), form a contiguous adhesive strip.

15. Adapter device according to one of the preceding claims, **characterized in that** the medical appliance (2a, 2b, 2c) is an administration device which can be secured on the body and which is used for medical administration of a liquid substance.

16. Adapter device according to one of the preceding claims, **characterized in that** the medical appliance is an insulin pump that can be secured on the human body.

17. Adapter device according to one of the preceding claims, **characterized in that** the medical appliance is a device which can be secured on the skin surface and which has at least one sensor for determining at least one physical condition.

## Revendications

1. Dispositif d'adaptateur pour l'application manuelle d'un appareil médical (2a, 2b, 2c) préparé avec un dispositif de collage (2d) sur la surface de la peau (3) d'un corps humain ou animal, comprenant un adaptateur (1) et un appareil médical (2), l'adaptateur (1) formant dans l'état initial une unité avec l'appareil médical présentant un dispositif de poignée (1a) et le dispositif d'adaptateur étant pourvu d'un mécanisme de pivotement (2c) qui divise l'appareil médical en une partie de pose (2a) et une partie pivotante (2b), **caractérisé en ce que** la partie de pose (2a) ainsi que la partie pivotante (2b) présentent un côté inférieur adhésif pour l'application de l'appareil (2a, 2b, 2c) sur la surface de la peau (3).

2. Dispositif d'adaptateur selon la revendication 1, **caractérisé en ce que** le mécanisme de pivotement (2c) permet une déviation de la partie pivotante (2b) suivant un angle qui, lorsque la partie de pose (2a) est appliquée sur la surface de la peau (3), empêche dans la position déviée un contact adhésif de la partie pivotante (2b) avec la surface de la peau.

3. Dispositif d'adaptateur selon la revendication 1 ou 2, **caractérisé en ce que** le mécanisme de pivotement (2c) coopère avec au moins un moyen de fixation (1b), qui permet, selon une solution de fixation, un rabattement de la partie pivotante (2b) d'une position déviée dans une position d'utilisation.

4. Dispositif d'adaptateur selon la revendication 3, **caractérisé en ce que** le mécanisme de pivotement (2c) est réalisé de manière constructive en tant qu'articulation avec une position d'encliquetage qui forme un moyen de fixation (1b).

5. Dispositif d'adaptateur selon la revendication 3 ou 4, **caractérisé en ce que** le moyen de fixation (1b) contient une zone destinée à la rupture qui permet, en cas de dépassement du couple agissant sur le mécanisme de pivotement (2c), un pivotement de la position fixée déviée dans la position d'utilisation.

6. Dispositif d'adaptateur selon la revendication 3, **caractérisé en ce que** le moyen de fixation (1b) présente une zone destinée à la rupture qui, en cas de dépassement d'une force de cisaillement agissant sur la zone destinée à la rupture, est sectionnée et provoque un pivotement de la position de déviation fixée dans la position d'utilisation.

7. Dispositif d'adaptateur selon la revendication 3, **caractérisé en ce que** le moyen de fixation (1b) est formé par une nervure amovible qui, une fois enlevée, provoque un pivotement de la partie pivotante (2b) de la position de déviation fixée dans la position d'utilisation.

8. Dispositif d'adaptateur selon l'une quelconque des revendications précédentes, **caractérisé en ce que** l'adaptateur est connecté dans l'état initial par engagement par force avec l'appareil médical (2a, 2b, 2c).

9. Dispositif d'adaptateur selon l'une quelconque des revendications précédentes, **caractérisé en ce que** l'adaptateur (1) peut être enlevé au moins en partie de l'appareil médical (2a, 2b, 2c) après réalisation de l'opération de fixation.

10. Dispositif d'adaptateur selon l'une quelconque des revendications 1 à 8, **caractérisé en ce que** le dispositif de poignée (1a) peut être reçu par l'appareil médical après la réalisation de l'opération de fixation.

11. Dispositif d'adaptateur selon l'une quelconque des revendications 1 à 10, **caractérisé en ce que** le mécanisme de pivotement (2c) est intégré dans le dispositif d'adaptateur dans le cas où un appareil médical de faible rigidité en flexion est utilisé.

12. Dispositif d'adaptateur selon l'une quelconque des revendications 1 à 10, **caractérisé en ce que** le mécanisme de pivotement (2b) est intégré dans l'appareil médical.

13. Dispositif d'adaptateur selon l'une quelconque des revendications précédentes, **caractérisé en ce que** les moyens adhésifs (2d) de la partie de pose (2a) et de la partie pivotante (2b), appliqués sur le côté inférieur de l'appareil, peuvent être arrachés indépendamment l'un de l'autre sous forme de rubans adhésifs.

14. Dispositif d'adaptateur selon l'une quelconque des revendications précédentes, **caractérisé en ce que** les moyens adhésifs (2d) de la partie de pose (2a) et de la partie pivotante (2b), appliqués sur le côté inférieur de l'appareil, forment un ruban adhésif continu.

15. Dispositif d'adaptateur selon l'une quelconque des revendications précédentes, **caractérisé en ce que** l'appareil médical (2a, 2b, 2c) est un dispositif d'administration pouvant être fixé sur le corps pour administrer une substance médicale fluide.

16. Dispositif d'adaptateur selon l'une quelconque des revendications précédentes, **caractérisé en ce que** l'appareil médical est une pompe à insuline pouvant être fixée sur le corps humain.

17. Dispositif d'adaptateur selon l'une quelconque des revendications précédentes, **caractérisé en ce que** l'appareil médical est un dispositif pouvant être fixé sur la surface de la peau et comprenant au moins un capteur pour détecter au moins un état du corps.
